# EUROPEAN PATENT SPECIFICATION

(11) **EP 2 771 328 B1**
(45) Date of publication and mention of the grant of the patent: **30.12.2015**
(21) Application number: 12780169.4
(22) Date of filing: 25.10.2012
(51) Int. Cl.: C07D 275/04

(54) **PROCESS FOR PREPARING 1,2-BENZISOTHIAZOLIN-3-ONE**
VERFAHREN ZUR HERSTELLUNG VON 1,2-BENZISOTHIAZOLIN-3-ON
PROCÉDÉ POUR PRÉPARER LA 1,2-BENZISOTHIAZOLIN-3-ONE

(30) Priority: 26.10.2011 US 201113281600
(43) Date of publication of application: 03.09.2014
(73) Proprietor: Titan Chemicals Limited, Road Town, 1110 Tortola (VG)
(72) Inventor: Berg, Carsten, 4791 Borre (DK); HOULLE-MARE, Didier, West Malling, Kent ME196RD (GB); SINGH, Sangita, Caterham, Surrey CR3 5EH (GB)
(74) Representative: Smith, Julian Philip Howard
(86) International application number: PCT/EP2012/071130
(87) International publication number: WO 2013/060766

(56) References cited:
- CA-A1- 1 269 985
- JP-A- 6 345 723
- SIEGEMUND A ET AL: "1,2-Benzisothiazol-3(2H)-Ones and Heterocyclic Annelated Isothiazol-3(2H)-Ones, Part II: Synthesis, Reactions, and Biological Activity", SULFUR REPORTS, HARWOOD ACADEMIC PUBLISHERS, CHUR, CH, vol. 23, no. 3, 1 January 2002 (2002-01-01), pages 279-319, XP009165054, ISSN: 0196-1772
- MASAO SHIMIZU ET AL: "Synthesis of 1,2-Benzisothiazolin-3-One by Transamination of Sulfenamides", HETEROCYCLES, ELSEVIER SCIENCE PUBLISHERS B.V. AMSTERDAM, NL, vol. 60, no. 8, 1 January 2003 (2003-01-01), pages 1855-1864, XP001526085, ISSN: 0385-5414

## Description

This invention relates to processes for making 1,2-benzisothiazolin-3-one which is sometimes known as "BIT" and has CAS# 2634-33-5. BIT is a commercially important biocide.

CA 1 269 985 and US 4 727 188 describe processes for preparing 1,2-benzisothiazolin-3-ones. In a first step and as described in US 4 727 188 anthranilamide is nitrosated by reaction with a nitrite (ie nitrate III) followed by reaction with sulfur dioxide to give 2,2'-dithiodibenzamide. It is well known that nitrosation is implicated in the formation of nitrosoamines which can be a health hazard and the reaction is in any event difficult to perform on an industrial scale. As an alternative to this reaction 2,2'-dithiodibenzamides can be made from the corresponding acyl chloride but according to US 4 727 188 this reaction is difficult to perform.

The ensuing 2,2' dithiodibenzamide is then subjected to oxidative ring closure. The reaction is performed in alkaline conditions in the presence of oxygen or an oxygen donor such as a peracid as described in CA 1 269 985.

The invention seeks therefore to provide alternative process for the production of 1,2-benzisothiazolin-3-one and salts thereof. It has now surprisingly been found that BIT and salts thereof can be produced in good yield by reaction of an alkali metal salt or ammonium salt (including mono-, di-, tri- and tetra-alkylammonium salts) of the corresponding 2-mercaptobenzamide or free 2-mercaptobenzamide with an oxidizing agent such as aqueous hydrogen peroxide.

According to the invention there is provided a process for preparing a 1,2-benzisothiazolin-3-one or a salt thereof comprising subjecting 2-mercaptobenzamide or a salt thereof to oxidative cyclisation. In some embodiments the salt of 2-mercaptobenzamide is the sodium salt. The 2-mercaptobenzamide or salt thereof can be subjected to oxidative cyclisation by reaction with aqueous hydrogen peroxide. The aqueous hydrogen peroxide can have a concentration in the range 8-30wt%. The invention further provides a process for preparing 1,2-benzisothiazolin-3-one or the sodium salt thereof comprising the steps of
i) heating a mixture of sodium sulfide hydrate and N-methyl 2-pyrrolidone
ii) distilling from the mixture water and optionally at least a portion of the N-methyl 2-pyrrolidone to leave water-depleted sodium sulfide and optionally water and/or N-methyl 2 pyrrolidone,
iii) adding at least one benzamide substituted at the 2-position selected from the group consisting of 2-chlorobenzamide, 2-fluorobenzamide, 2-nitrobenzamide, 2-cyanobenzamide and 2-C₁₋₆ branched or straight chain alkoxybenzamide to the water-depleted sodium sulfide to give a composition comprising 2-mercaptobenzamide or the sodium salt thereof and
iv) subjecting the composition comprising 2-mercaptobenzamide or salt thereof to oxidative cyclisation.

2-Mercaptobenzamide is a known material. It is described for example in JP06345723. According to that document 2-mercaptobenzamide can be made by the reaction of 2-halobenzamide with 60% sodium sulfide. The authors of that document contend that when 2-chlorobenzamide is used as the starting material the yield of 2-mercaptobenzamide is 85%. No information of the purity of the 2-mercaptobenzamide is given. The present inventors have repeated the process set forth in Example 1 of JP06345723. The results are set forth herein and show that the yield of pure 2-mercaptobenzamide is low. The purity of the obtained 2-mercaptobenzamide was 56% and the yield based on 2-chlorobenzamide was 43%. The present invention flows from the present inventors' realisation that the large by-product fraction is due to the use of 60% sodium sulfide. The remainder of the crude sodium sulfide is water which hydrolyses the amide functionality of the benzamide.

Commercially available sodium sulfide is available only as the hydrate and contains considerable water. It is not available in industrially useful amounts in anhydrous form. It is possible to produce anhydrous sodium sulphide in the laboratory by reaction of sodium with sulfur in liquid ammonia but this technique is too expensive for industrial scale use.

In accordance with an aspect of the invention in an initial step a sodium sulfide hydrate which is usually obtained in the form of lumps, flakes or powder is slurried with N-methyl-2-pyrrolidone (hereinafter sometimes referred to as "NMP") and heated in a dry atmosphere for example under dry nitrogen until at least some of the water is evaporated optionally with some of the N-methyl-2-pyrrolidone. Typical heating temperatures are between the boiling point of water and the boiling point of N-methyl-2-pyrrolidone at the pressure at which the transformation is taking place. At ambient pressure this implies a temperature in the range of about 100 to about 200°C preferably the temperature is about 150, 160 or 170 to about 190 or 200°C. The process is not restricted to ambient pressure and other pressures either higher or lower may be used. In preferred embodiments the process is performed at reduced pressure such as 50 to 850 hPa (mbar), in particular from 100 to 400 hPa, preferably from 150 to 350 hPa since less energy is required than at atmospheric pressure.

Other materials which are compatible with sodium sulfide such as dimethyl sulfoxide, dimethylformamide, dioxane, hexamethylphosphorotriamide, 2-methyltetrahydrofuran, sulfolane, acetamide, ethylene carbonate, propylene carbonate, ethanol, acetonitrile, butanol especially n-butanol, cyclohexanol, 1,3-dimethyl-3,4,5,6-tetrahydro-2(1H)-pyrimidone ("DMPU"), dimethylacetamide or 2-pyrrolidone or mixtures thereof can be used. These materials are compatible with sodium sulphide and have a higher boiling point than water and/or form azeotropes with water. In general the best results are obtained using N-methyl-2-pyrrolidone. Some materials allow use of Dean-Stark type apparatus in which water is collected for disposal and the other materials are recycled. The dried sodium sulfide can be recovered but preferably is used directly for further reaction.

The dried sodium sulfide is then reacted with 2-chlorobenzamide. It is found that much less 2-chlorobenzoic acid is formed compared to when untreated sodium sulfide is used. Additionally next to no starting material remains unreacted. Additionally a small amount of 2,2'-dithiodibenzamide is formed in addition to the 2-mercaptobenzamide. This is not a problem since 2,2'-dithiodibenzamide itself undergoes oxidative cyclization to form BIT. Similar results are obtained using other 2-halobenzamides such as 2-bromobenzamide and especially 2-fluorobenzamide.

In place of sodium sulfide, other sulfides (SH⁻) can be used especially those of potassium, lithium and ammonium. These materials are generally anhydrous and so it is not necessary to conduct the drying step.

In principle mercaptobenzamide can be isolated either in free form or as a salt and purified but in practice it is not often necessary to isolate the product in pure form. The 2-mercaptobenzamide or salt thereof can then be oxidatively cyclised for example using hydrogen peroxide to give 1,2-benzisothiazolin-3-one or its salts. Other suitable reagents for oxidative cyclisation may include molecular oxygen for example air, ozone, sodium chlorate (I), sodium perborate, sodium percarbonate, sodium perphosphate, potassium permanganate, ruthenium tetroxide, osmium tetroxide and organic peroxides such as MCPBA, peracetic acid, perbenzoic acid, and perphthalic acid. The preferred oxidative cyclisation reagent is however an aqueous solution of hydrogen peroxide. Preferably the aqueous solution of hydrogen peroxide contains less than about 68wt% hydrogen peroxide on safety grounds. Suitable concentrations may be in the range of about 3wt% to about 68wt% for example about 6wt% to about 30wt% such as about 10 to 20wt% for example about 14wt%.

In some embodiments the oxidative cyclisation is performed in N-methyl-2-pyrrolidone. Other solvents such as dimethyl sulfoxide, dimethylformamide, dioxane, hexamethylphosphorotriamide, tetrahydrofuran, water, alcohols such as methanol or ethanol, ketones such as acetone and methyl ethyl ketone, esters such as ethyl acetate, sulfolane, 2-pyrrolidone, 1,2-dimethyl imidazole, 1,3-dimethylimidazolidine, dimethyl sulfone, 1,3-dimethyl-3,4,5,6-tetrahydro-2(1H)-pyrimidone ("DMPU"), dimethylacetamide and acetamide can be used.

The hydrogen peroxide solution may function as a solvent and in some embodiments it is not necessary to add further solvent. Particularly good results are however achieved with dimethyl sulfoxide but N-methyl-2-pyrrolidone also gives good results.

Typically the reaction mixture containing the at least partially dried sodium sulfide is cooled to about 130°C for example in the range about 120 to about 160°C and then the substituted benzamide compound such as chlorobenzamide is added. The mixture is then allowed to react. In order to speed the reaction it may be desirable to heat the mixture for example to a temperature in the range about 150 to about 190°C. If wished it is possible to monitor progress of the reaction by for example HPLC. Those skilled will have little difficulty in selecting suitable analytical techniques.

When the reaction is sufficiently advanced the mixture is allowed to cool.

In some embodiments any excess sodium sulfide can be destroyed by adding a mineral acid such as hydrochloric acid and boiling until hydrogen sulfide evolution ceases.

This however is not preferred since the evolved highly toxic hydrogen sulfide has to be disposed of. In preferred embodiments the hydrogen sulfide is destroyed in situ for example by an excess of the oxidizing agent such as hydrogen peroxide, dimethyl sulfoxide and mixtures thereof.

Aqueous hydrogen peroxide is added slowly and once all the peroxide has been added the mixture is worked up by distilling off the water and N-methyl pyrrolidone.

The 1,2-benzisothiazolin-3-one salt can be removed from the reaction mixture for use or can be converted to the 1,2-benzisothiazolin-3-one by reaction with an acid such as hydrochloric acid.

While 2-chlorobenzamide is preferred on cost grounds to produce the 2-mercaptobenzamide it is also possible to use 2-fluorobenzamide, 2-bromobenzamide or other benzamides having an electron withdrawing group at the 2-position such as nitro-, cyano-, sulfonate such as sulfonic acid, sulfonate ester such as tosyl, mesyl and benzenesulfonyl, carbonyl groups such as carboxylic acid and ester functionality such as - COOR where R is C₁₋₆ branched or straight chain alkyl, trichloromethyl or trifluoromethyl and -OR where R is C₁₋₆ branched or straight chain alkyl. 2-Fluorobenzamide and 2-chlorobenzamide are especially preferred.

Preferred embodiments of the invention are one-pot reactions in which initially sodium sulfide hydrate is heated with the polar aprotic solvent until sufficient water has been driven off (for example as determined by weight loss) then adding the benzamide and after a further period cooling the mixture and subjecting it to oxidative cyclisation.

Commercial sodium sulfide generally is supplied as the hydrate, Na₂S.xH₂O, where the percentage of Na₂S is specified. It is therefore a straightforward matter to calculate how much water is present in the sodium sulfide hydrate. Desirably it is heated with the N-methyl 2-pyrrolidone until much for example at least 50%, more preferably at least 60% such as at least 70% of the water for example at least 80% or at least 90% or even 100% or substantially all of the water is driven off. This can be determined by weighing, by analytical techniques known to the skilled in the art or by spectroscopy. Alternatively the mixture can simply be heated for a period known by experiment or experience to be long enough.

### Example 1 (Comparative)

The process for synthesis of 2-mercaptobenzamide, set forth in example 1 of JP 06 345723 was repeated:
15.6 g (0.0983 mol) of 98% 2-chlorobenzamide, 16.0g (0.1230 mol) of 60% sodium sulfide and 100g NMP were added to a 200ml 3 neck flask provided with a stirrer, heated oil bath thermometer and condenser. The mixture was stirred at 160°C for 4 hours.
NMP was distilled off at reduced pressure and the residue dissolved in 100g of water.

The mixture was acidified to pH 2.0 by adding 35% hydrochloric acid at 20°C.

The separated crystalline material was isolated by filtration, washed with water and dried to constant weight of 11.6 g at 40°C.

HPLC analysis, calibrated by authentic samples, revealed the following composition of the isolated product:
(g; mol; molar% of theory): (1.2; 0.0071; 7.2) 2-chlorobenzamide, (2.8; 0.0177; 18.0) 2-chlorobenzoic acid and (6.5; 0.0424; 43.2) 2-mercaptobenzamide.

Based on weight the purity of 2-mercaptobenzamide is 56.0%.

### Example 2

23.4g (0.18 mol) of 60% sodium sulfide (40% water) and 160g N-methyl pyrrolidone (NMP) was added to a 500ml 3 neck flask provided with a heated oil bath, stirrer and a thermometer. The mixture was stirred at 190°C and purged with nitrogen to a weight loss of 25g. To the dried slurry of sodium sulfide at 130°C, 18.1g (0.1163 mol), 2-chlorobenzamide of 98% purity was added and the mixture heated to 175°C for 4 hours. HPLC analysis of the reaction mixture, calibrated with authentic samples showed (g; mol; molar % of theory): (0.37; 0.0012; 2) 2,2'-dithiodibenzoic acid and (16.5; 0.108; 88) 2-mercaptobenzamide. 2-chlorobenzoic acid was not detected.

The mixture was cooled to 70°C, 40g of water was added and pH adjusted to 4.0, by adding 28.5g of 35% hydrochloric acid. The mixture was heated to boiling until hydrogen sulfide evolution ceased. Evolved hydrogen sulfide was absorbed for disposal in a caustic solution. At 20°C, caustic solution (such as alkali hydroxide or carbonates or other salts or other bases) was added to the reaction mixture to bring the pH back to 9 or above and 27.0g (0.111 mol) 14% hydrogen peroxide was introduced over 30 min.

Water and NMP was distilled off at reduced pressure and the residue was dispersed in 125g of water. The mixture was adjusted to pH5with 35% hydrochloric acid. Separated BIT crystals were filtered off, washed with water and air dried to constant weight. BIT yield 14.3g (0.094 mol) which is 80.8% of theory. Purity 99.5% by HPLC. The NMP can be recovered for reuse by known methods.

### Example 3

23.4g (0.12 mol) of 60% sodium sulfide (40% water) and 160g N-methylpyrrolidone (NMP) was added to a 500ml 3 neck flask provided with a heated oil bath, stirrer and a thermometer. The mixture was stirred at 190°C and purged with nitrogen to a weight loss of 25g. To the dried slurry of sodium sulfide at 130°C, 18.1g (0.117 mol), 2-chlorobenzamide was added and the mixture heated to 175°C for 4 hours. HPLC analysis of the reaction mixture, calibrated with authentic samples showed (g; mol; molar % of theory): (0.37; 0.0012; 2) 2,2'-dithiodibenzoic acid and (16.5; 0.108; 88) 2-mercaptobenzamide. 2-chlorobenzoic acid was not detected. NMP was distilled off at reduced pressure.

The mixture was cooled to 70°C, 125g of water was added and pH adjusted to 3.0, by adding 28.5g of 35% hydrochloric acid. The mixture was heated to boiling until hydrogen sulfide evolution ceased. Instead of boiling hydrogen sulfide evolution could be promoted by a gas stream. Evolved hydrogen sulfide can be absorbed in a caustic solution for disposal. At 20°C, caustic solution (such as alkali hydroxide or carbonate or other salts or other bases) is added to the reaction mixture to bring the pH back to 9 or above. 27.0g (0.111 mol) 14% hydrogen peroxide was introduced over 30 min.

The mixture was acidified to pH 5with 35% hydrochloric acid and the BIT crystals filtered off, washed with water and air dried to constant weight. The yield and quality of BIT was the same as in Example 2.

### Example 4

23.4g (0.12 mol) of 60% sodium sulfide (40% water) and 160g N-methylpyrollidone (NMP) was added to a 500ml 3 neck flask provided with a heated oil bath, stirrer and a thermometer. The mixture was stirred at 130°C and water followed by water/NMP, then NMP is distilled off under vacuum until about 10wt% of the total reaction mixture was distilled off. The oil bath temperature was adjusted to maintain distillation. The reaction mixture was cooled down to 130°C. To the dried slurry of sodium sulfide at 130°C, 18.1g (0.117 mol), 2-chlorobenzamide (which can be dissolved in NMP or other inert organic solvent such as aprotic polar solvent) was added and the mixture heated to 175°C for 4 hours under nitrogen. HPLC analysis of the reaction mixture, calibrated with authentic samples showed completion of reaction (<0.5% of starting material. 2-Chlorobenzoic acid was not detected.)

At 20°C, caustic solution (such as alkali hydroxide or salts such as carbonates or other bases) was added to the reaction mixture to bring the pH to 9 or above. 27.0g (0.111 mol) 14% hydrogen peroxide was introduced over 30 min and the mixture stirred until completion of reaction. If necessary more hydrogen peroxide was added. The solvents (water and NMP) were distilled off under vacuum and the residue is taken back in water. The aqueous mixture was acidified to pH 4 with 35% hydrochloric acid and the BIT crystals were filtered off, washed with water and air dried to constant weight.

### Example 5

Example 4 was repeated up to the point where the reaction of 2-chlorobenzamide with sodium sulfide was complete.

The solvent (NMP) was distilled off under vacuum and the residue taken back in water.

At 20°C, caustic solution (such as alkali hydroxide or carbonate or other salts or other bases) was added to the reaction mixture to bring the pH to 9 or above.27.0g (0.111 mol) 14% hydrogen peroxide was introduced over 30 min. The reaction mixture was stirred until completion of reaction if necessary with the addition of further hydrogen peroxide. The aqueous solution was acidified to pH 5 with 35% hydrochloric acid and BIT crystals filtered off, washed with water and air dried to constant weight.

### Example 6

Example 4 was repeated until the reaction of 2-chlorobenzamide with sodium sulfide was complete.

The solvent (NMP) was distilled off under vacuum and the residue treated with DMSO.

At 20°C, potassium carbonate (or alkali hydroxide or other base) was added to the reaction mixture to bring the pH to 9 or above. 27.0g (0.111 mol) 14% hydrogen peroxide was introduced over 30 min and the mixture stirred until reaction was complete. If necessary more hydrogen peroxide was added. DMSO was distilled off under vacuum and the residue was stirred with toluene for 30 minutes. The solid was decanted and rinsed with toluene. It was then treated with water and then acidified to pH 5 with 35% hydrochloric acid. BIT crystals were filtered off, washed with water and air dried to constant weight.

### Example 7

Example 4 was repeated until the reaction of 2-chlorobenzamide with sodium sulfide was complete save that 7.4g (0.0569 mol) of 60% sodium sulfide (40% water), 133g (NMP) and 6.0g (0.03856 mol), 2-chlorobenzamide were employed.

NMP was distilled off under vacuum and the residue was taken back in water.

The reaction mixture was acidified to pH 6.0 with concentrated HCl, and then potassium carbonate powder was added to bring the pH to 10.5. Hydrogen peroxide was added to the reaction mixture. The ensuing reaction was followed by HPLC sampling and once substantially complete the reaction mixture was acidified to pH4.5 at temperature <10°C resulting in precipitation of BIT. The reaction mixture was stirred for at least 1 hour at <10°C, then the BIT was filtered under vacuum. The cake was washed with water and dried. Yield >80% purity>97%.

### Example 8

Example 4 was repeated save that in place of 2-chlorobenzamide 16.3g (0.117 mol), 2-fluorobenzamide was used. The reaction was substantially faster than when 2-chlorobenzamide was used and was complete in 65 minutes at 170°C as compared with 240 minutes at 175°C when 2-chlorobenzamide was used.

## Claims

1. A process for preparing 1,2-benzisothiazolin-3-one or the sodium salt comprising the steps of
i) heating a mixture of sodium sulfide hydrate and N-methyl 2-pyrrolidone
ii) distilling from the mixture water and optionally at least a portion of the N-methyl 2-pyrrolidone to leave water-depleted sodium sulfide and optionally water and/or N-methyl 2 pyrrolidone,
iii) adding at least one benzamide substituted at the 2-position selected from the group consisting of 2-chlorobenzamide, 2-fluorobenzamide, 2-nitrobenzamide, 2-cyanobenzamide and 2-C₁₋₆ branched or straight chain alkoxybenzamide to the water-depleted sodium sulfide to give a composition comprising 2-mercaptobenzamide or the sodium salt thereof and
iv) subjecting the composition comprising 2-mercaptobenzamide or salt thereof to oxidative cyclisation.

2. A process as claimed in claim 1 wherein the 2-mercaptobenzamide or sodium salt thereof is subjected to oxidative cyclisation by reaction with aqueous hydrogen peroxide.

3. A process as claimed in claim 2 wherein the aqueous hydrogen peroxide has a concentration in the range 8-30wt%.

## Patentansprüche

1. Verfahren zur Herstellung von 1,2-Benzisothiazolin-3-on oder des Natriumsalzes, umfassend die folgenden Schritte
i) Erwärmen eines Gemisches aus Natriumsulfidhydrat und N-Methyl-2-pyrrolidon,
ii) Destillieren von Wasser und optional mindestens einem Teil des N-Methyl-2-pyrrolidons aus dem Gemisch, um an Wasser abgereichertes Natriumsulfid und optional Wasser und/oder N-Methyl-2-pyrrolidon zu hinterlassen,
iii) Hinzufügen von mindestens einem an der 2-Position substituierten Benzamid, das aus der Gruppe ausgewählt wird, die sich aus 2-Chlorbenzamid, 2-Fluorbenzamid, 2-Nitrobenzamid, 2-Cyanobenzamid und verzweigt- oder geradkettigem 2-C₁₋₆-Alkoxybenzamid zusammensetzt, zu dem an Wasser abgereicherten Natriumsulfid, um eine Zusammensetzung zu erhalten, umfassend 2-Mercaptobenzamid oder das Natriumsalz davon und
iv) Unterziehen der Zusammensetzung, umfassend 2-Mercaptobenzamid oder das Salz davon, einer oxidativen Zyklisierung.

2. Verfahren nach Anspruch 1, wobei das 2-Mercaptobenzamid oder das Natriumsalz davon einer oxidativen Zyklisierung durch eine Reaktion mit wässrigem Wasserstoffperoxid unterzogen wird.

3. Verfahren nach Anspruch 2, wobei das wässrige Wasserstoffperoxid eine Konzentration in dem Bereich von 8-30 Gew.-% aufweist.

## Revendications

1. Procédé de préparation de 1,2-benzisothiazolin-3-one ou du sel de sodium, comprenant les étapes suivantes
i) le chauffage d'un mélange contenant du sulfure de sodium hydraté et de la N-méthyl-2-pyrrolidone,
ii) la distillation, à partir du mélange, de l'eau et éventuellement d'au moins une partie de la N-méthyl-2-pyrrolidone pour laisser du sulfure de sodium appauvri en eau et éventuellement de l'eau et de la N-méthyl-2-pyrrolidone,
iii) l'ajout d'au moins un benzamide substitué en position 2 choisi dans le groupe constitué du 2-chlorobenzamide, du 2-fluorobenzamide, du 2-nitrobenzamide, du 2-cyanobenzamide et d'un 2-(alcoxy en C₁ à C₆ linéaire ou ramifié) benzamide, au sulfure de sodium appauvri en eau pour obtenir une composition comprenant du 2-mercaptobenzamide ou son sel de sodium, et
iv) la soumission de la composition comprenant du 2-mercaptobenzamide ou son sel à une cyclisation oxydative.

2. Procédé selon la revendication 1, dans lequel le 2-mercaptobenzamide ou son sel de sodium est soumis à une cyclisation oxydative par réaction avec du peroxyde d'hydrogène aqueux.

3. Procédé selon la revendication 2, dans lequel le peroxyde d'hydrogène aqueux présente une concentration située dans la plage allant de 8 % à 30 % en poids.
